# EUROPEAN PATENT SPECIFICATION

(11) **EP 1 173 436 B1**
(45) Date of publication and mention of the grant of the patent: **09.06.2004**
(21) Application number: 00920893.5
(22) Date of filing: 19.04.2000
(51) Int. Cl.: C07D 417/12, A61K 31/427, A61P 3/10

(54) **THIAZOLIDINEDIONE DERIVATIVE AND ITS USE AS ANTIDIABETIC**
THIAZOLIDINDIONDERIVAT UND SEINE VERWENDUNG ALS ANTIDIABETIKUM
DERIVE DE THIAZOLIDINEDIONE ET SON UTILISATION COMME ANTIDIABETIQUE

(30) Priority: 20.04.1999 GB 9909041
(43) Date of publication of application: 23.01.2002
(60) Divisional application: 03079073.7
(73) Proprietor: SmithKline Beecham plc, Brentford, Middlesex TW8 9GS (GB)
(72) Inventor: Blackler, Paul David James, SmithKline Beecham, Tonbridge, Kent TN 11 9AN (GB); Giles, Robert Gordon, SmithKline Beecham Phar., Tonbridge, Kent TN11 9AN (GB); SASSE, Michael John, SmithKline Beecham Phar., Tonbridge, Kent TN11 9AN (GB)
(74) Representative: Rutter, Keith
(86) International application number: PCT/GB2000/001521
(87) International publication number: WO 2000/063205

(56) References cited:
- WO-A-94/05659
- CANTELLO B C C ET AL: "Facile biocatalytic reduction of the carbon-carbon double bond of 5-benzylidenethiazolidine-2,4-diones. Synthesis of (+,-)-5-(4-{2-[methyl(2-pyridylamino] ethoxy}benzyl)thiazolidine-2,4 -dione (BRL49653), its (R)-(+)-enantiomer and analogues" JOURNAL OF THE CHEMICAL SOCIETY, PERKIN TRANSACTIONS 1, 1994, pages 3319-3324-3322, XP002099539 ISSN: 0300-922X
- HALEBLIAN J ET AL: "Pharmaceutical application of polymorphism" JOURNAL OF PHARMACEUTICAL SCIENCES, vol. 58, no. 8, 1 August 1969 (1969-08-01), pages 911-929, XP002020518 ISSN: 0022-3549

## Description

This invention relates to a novel pharmaceutical, to a process for the preparation of the pharmaceutical and to the use of the pharmaceutical in medicine.

European Patent Application, Publication Number 0,306,228 relates to certain thiazolidinedione derivatives disclosed as having hypoglycaemic and hypolipidaemic activity. The compound of example 30 of EP 0,306,228 is 5-[4-[2-(N-methyl-N-(2-pyridyl)amino)ethoxy]benzyl]thiazolidine-2,4-dione (hereinafter also referred to as "Compound I").

International Patent Application, Publication Number WO94/05659 discloses certain salts of the compounds of EP 0,306,228 and in particular the maleic acid salt.

International Patent Applications, Publication Numbers WO99/31093, WO99/31094 and WO99/31095 each disclose distinct hydrates of Compound (I).

It has now been discovered that Compound (I) exists in the form of a novel hydrochloride salt which is dihydrated. This novel hydrochloride salt dihydrate (hereinafter also referred to as "Hydrochloride Hydrate") is particularly suitable for bulk preparation and handling. The novel form can be prepared by an efficient, economic and reproducible process particularly suited to large-scale preparation.

The novel Hydrochloride Hydrate also has useful pharmaceutical properties and in particular it is indicated to be useful for the treatment and/or prophylaxis of diabetes mellitus, conditions associated with diabetes mellitus and certain complications thereof.

Accordingly, the present invention provides 5-[4-[2-(N-methyl-N-(2-pyridyl)amino)ethoxy]benzyl]thiazolidine-2,4-dione, hydrochloride dihydrate characterised in that it:
(i) provides an infrared spectrum containing peaks at 3392, 2739, 1751, 1325 and 713 cm⁻¹; and/or
(ii) provides an X-ray powder diffraction (XRPD) pattern containing peaks at 9.1, 12.0, 15.7, 16.3 and 19.8 °2θ.

In one favoured aspect, Hydrochloride Hydrate provides an infra red spectrum substantially in accordance with Figure I, which provides infra red peak positions at: 3392, 3139, 3094, 2739, 1758, 1751, 1706, 1643, 1632, 1610, 1583, 1545, 1513, 1412, 1357, 1325, 1297, 1265, 1251, 1216, 1179, 1152, 1138, 1110, 1080, 1053, 1033, 1010, 985, 953, 931, 909, 827, 822, 812, 769, 739, 724, 713, 660, 620, 604, 593, 562, 539, 529 and 508 cm⁻¹.
In one favoured aspect, Hydrochloride Hydrate provides an X-Ray powder diffraction pattern (XRPD) substantially in accordance with Figure II, which provides peaks at 9.1, 12.0,, 15.7, 16.3, 18.2, 18.6, 19.8, 20.9, 21.6, 22.8, 24.1, 24.7, 25.4, 26.0, 27.5, 27.7, 28.7, 31.4, 31.6, 32.1, 33.1, 33.6 and 34.6 °2θ.

The Hydrochloride Hydrate may exist in certain dehydrated forms which reversibly convert to the Hydrochloride Hydrate when contacted with water, either in liquid or vapour form. The present invention encompasses all such reversibly rehydratable forms of the Hydrochloride Hydrate. Preferably, there is provided the hydrated form as characterised above.

The present invention encompasses Hydrochloride Hydrate isolated in pure form or when admixed with other materials.

Thus in one aspect there is provided Hydrochloride Hydrate in isolated form.

In a further aspect there is provided Hydrochloride Hydrate in pure form.

In yet a further aspect there is provided Hydrochloride Hydrate in crystalline form.

The invention also provides a process for preparing Hydrochloride Hydrate, characterised in that 5-[4-[2-(N-methyl-N-(2-pyridyl)amino)ethoxy]benzyl]thiazolidine-2,4-dione (Compound I) is treated with aqueous hydrochloric acid and thereafter the required compound is recovered.

Suitably, the aqueous hydrochloric acid is formed by admixing concentrated hydrochloric acid and water or an appropriate organic solvent, such as denatured ethanol or propan-2-ol, or mixtures thereof.

Suitably the reaction is carried out at ambient temperature but any convenient temperature may be employed which provides the required product.

In one aspect the Hydrochloride Hydrate is prepared by:
(i) treating 5-[4-[2-(N-methyl-N-(2-pyridyl)amino)ethoxy]benzyl]thiazolidine-2,4-dione in aqueous propan-2-ol with aqueous hydrochloric acid at an elevated temperature, such as a temperature in the range of from 60°C to 80°C, suitably 65°C to 75 °C, for example 75°C;
(ii) the solution is cooled to ambient temperature, for example 25°C;
(iii) the solution is seeded with the Hydrochloride Hydrate, preferably the reaction temperature is further reduced to a temperature in the range of from 0 to 5°C to complete crystallisation.

In the above mentioned process the total amount of water in the reaction mixture is preferably in the range of from 15% to 50%w/v, suitably 25% to 40%w/v, for example 27%w/v.

It will be appreciated that in the above mentioned processes hydrochloric acid may be replaced by any suitable source of hydrochloride ions, providing the amount of water in the reaction is suitable for formation of the of Hydrochloride Hydrate. The suitable amount of water is generally at least two molar equivalents and generally an excess over this, for example an amount equivalent to that used in the above mentioned processes.

Recovery of the required compound generally comprises crystallisation from an appropriate solvent such as water or aqueous denatured ethanol.

Crystallisation is generally carried out at low to ambient temperature, such as in the range of from 0 to 30°C for example 25°C; alternatively crystallisation may be initiated at an elevated temperature, such as in the range of from 30°C and 60°C for example 50°C, and then completed by allowing the temperature of the solvent to cool to ambient or low temperature, such as in the range of from 0 to 30°C for example 25°C. The crystallisation can be initiated by seeding with crystals of Hydrochloride Hydrate but this is not essential.

Compound (I) is prepared according to known procedures, such as those disclosed in EP 0,306,228 and W094/05659.

When used herein the term 'prophylaxis of conditions associated with diabetes mellitus' includes the treatment of conditions such as insulin resistance, impaired glucose tolerance, hyperinsulinaemia and gestational diabetes.

Diabetes mellitus preferably means Type II diabetes mellitus.

Conditions associated with diabetes include hyperglycaemia and insulin resistance and obesity. Further conditions associated with diabetes include hypertension, cardiovascular disease, especially atherosclerosis, certain eating disorders, in particular the regulation of appetite and food intake in subjects suffering from disorders associated with under-eating , such as anorexia nervosa, and disorders associated with over-eating, such as obesity and anorexia bulimia. Additional conditions associated with diabetes include polycystic ovarian syndrome and steroid induced insulin resistance.

The complications of conditions associated with diabetes mellitus encompassed herein includes renal disease, especially renal disease associated with the development of Type II diabetes including diabetic nephropathy, glomerulonephritis, glomerular sclerosis, nephrotic syndrome, hypertensive nephrosclerosis and end stage renal disease.

As mentioned above the compound of the invention has useful therapeutic properties: The present invention accordingly Hydrochloride Hydrate for use as an active therapeutic substance.

More particularly, the present invention provides Hydrochloride Hydrate for use in the treatment and/or prophylaxis of diabetes mellitus, conditions associated with diabetes mellitus and certain complications thereof.

Hydrochloride Hydrate may be administered per se or, preferably, as a pharmaceutical composition also comprising a pharmaceutically acceptable carrier. The formulation of Hydrochloride Hydrate is generally as disclosed for Compound (I) in the above mentioned publications.

Accordingly, the present invention also provides a pharmaceutical composition comprising Hydrochloride Hydrate and a pharmaceutically acceptable carrier therefor.

Hydrochloride Hydrate is normally administered in unit dosage form.

The active compound may be administered by any suitable route but usually by the oral or parenteral routes. For such use, the compound will normally be employed in the form of a pharmaceutical composition in association with a pharmaceutical carrier, diluent and/or excipient, although the exact form of the composition will naturally depend on the mode of administration.

Compositions are prepared by admixture and are suitably adapted for oral, parenteral or topical administration, and as such may be in the form of tablets, capsules, oral liquid preparations, powders, granules, lozenges, pastilles, reconstitutable powders, injectable and infusable solutions or suspensions, suppositories and transdermal devices. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

Solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the active compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the active compound is suspended in the vehicle instead of being dissolved and sterilised by exposure to ethylene oxide before suspending in the sterile vehicle. Advantageously, a surfactant or wetting agent is included in the composition to facilitate uniform distribution of the active compound.

As is common practice, the compositions will usually be accompanied by written or printed directions for use in the medical treatment concerned.

As used herein the term 'pharmaceutically acceptable' embraces compounds, compositions and ingredients for both human and veterinary use: for example the term 'pharmaceutically acceptable salt' embraces a veterinarily acceptable salt.

The present invention further provides a method for the treatment and/or prophylaxis of diabetes mellitus, conditions associated with diabetes mellitus and certain complications thereof, in a human or non-human mammal which comprises administering an effective, non-toxic, amount of Hydrate to a human or non-human mammal in need thereof.

Conveniently, the active ingredient may be administered as a pharmaceutical composition hereinbefore defined, and this forms a particular aspect of the present invention.

In addition, the Hydrochloride Hydrate may be used in combination with other antidiabetic agents such as insulin secretagogues, for example sulphonylureas, biguanides, such as metformin, alpha glucosidase inhibitors, such as acarbose, beta agonists, and insulin such as those disclosed in WO98/57649, WO98/57634, WO98/57635 or W098/57636. The other antidiabetic agents, the amounts thereof and methods of administration are as described in the above mentioned publications. The formulation of the Hydrochloride Hydrate and dosages thereof in said combinations are generally as disclosed for Compound (I) in the above mentioned publications.

In a further aspect the present invention provides the use of Hydrochloride Hydrate for the manufacture of a medicament for the treatment and/or prophylaxis of diabetes mellitus, conditions associated with diabetes mellitus and certain complications thereof.

In the treatment and/or prophylaxis of diabetes mellitus, conditions associated with diabetes mellitus and certain complications thereof Hydrochloride Hydrate may be taken in doses, such as those described in the above mentioned publications

No adverse toxicological effects are indicated in the above mentioned treatments for the compounds of the invention.

The following examples illustrate the invention but do not limit it in any way.

### Preparation of 5-[4-[2-(N-methyl-N-(2-pyridyl)amino)ethoxy]benzyl]thiazolidine-2,4-dione, hydrochloride dihydrate

**Example 1:** 5-[4-[2-(N-methyl-N-(2-pyridyl)amino)ethoxy]benzyl]thiazolidine-2,4-dione (4 g) was suspended in water (25 ml), and concentrated hydrochloric acid (4 ml) was added, forming a clear solution, and then a thick suspension. After 1 hour the suspension was diluted with water (10 ml), the crude product was filtered and then washed with denatured ethanol (20 ml). The crude product was stirred in diethyl ether (50 ml), filtered and dried at 50°C to afford the title compound(3.1 g ,63%).

**Example 2:** 5-[4-[2-(N-methyl-N-(2-pyridyl)amino)ethoxy]benzyl]thiazolidine-2,4-dione (4 g) was suspended in denatured ethanol (40 ml), and concentrated hydrochloric acid (4 ml) was added, forming a solution. Water (40 ml) was added, and the resulting suspension was cooled to 0°C and stirred for 3 hours. The product was filtered, washed with acetone (2 x 10 ml) and dried at 50°C to afford the title compound- (3.91 g, 82%).

**Example 3:** A mixture of 5-[4-[2-(N-methyl-N-(2-pyridyl)amino)ethoxy]benzyl]thiazolidine-2,4-dione (5.0 g), propan-2-ol (75 ml) and water (20 ml) was stirred and heated to 75°C. Aqueous hydrochloric acid (2.8M, 10 ml) was added and the mixture stirred for 15 minutes at which point a clear solution was observed. After cooling to 25°C seed crystals of rosiglitazone hydrochloride dihydrate were added and the mixture cooled to 0-5°C and stirred for 2 hours. The product was collected by filtration, washed with propan-2-ol (20 ml) and dried under vacuum for 16 hours to give Hydrochloride Hydrate as a white crystalline solid (4.8 g).

**CHARACTERISING DATA:** The following characterising data were generated for Hydrochloride Hydrate:
**A Water content**
   This was determined as 8.4% w/w using a Karl Fischer apparatus (theory for dihydrate 8.37% w/w).
**B Ionic Chlorine**
   This was determined as 8.5% w/w (theory for dihydrate 8.26% w/w).
**C Infrared**
   The infrared absorption spectrum of a mineral oil dispersion of Hydrochloride Hydrate was obtained using a Nicolet 710 FT-IR spectrometer at 2 cm⁻¹ resolution. Data were digitised at 1 cm⁻¹ intervals. The spectrum obtained is shown in Figure I. Peak positions are as follows: 3392, 3139, 3094, 2739, 1758, 1751, 1706, 1643, 1632, 1610, 1583, 1545, 1513, 1412, 1357, 1325, 1297, 1265, 1251, 1216, 1179, 1152, 1138, 1110, 1080, 1053, 1033, 1410, 985, 953, 931, 909, 827, 822, 812, 769, 739, 724, 713, 660, 620, 604, 593, 562, 539, 529 and 508 cm⁻¹.

### B X-Ray Powder Diffraction (XRPD)

The XRPD pattern of Hydrochloride Hydrate is shown below in Figure II and a summary of the XRPD angles and calculated lattice spacings characteristic of Hydrochloride Hydrate is given in Table I.
A Bruker AXS D8 Advance X-ray powder diffractometer (Cu X-ray source) was used to generate the pattern using the following acquisition conditions:

| | |
|---|---|
| Tube anode | Cu |
| Generator tension | 40 kV |
| Generator current | 40 mA |
| Start angle | 2.0°2θ |
| End angle | 35.0°2θ |
| Step size | 0.02°2θ |
| Time per step | 2.5s |

**Table I.**

| X-Ray Powder Diffraction Angles and Calculated Lattice Spacing Characteristic of Hydrochloride Hydrate. | |
|---|---|
| Diffraction Angle (°2θ) | Lattice Spacing (Angstroms) |
| 9.1 | 9.75 |
| 12.0 | 7.37 |
| 15.7 | 5.64 |
| 16.3 | 5.42 |
| 18.2 | 4.88 |
| 18.6 | 4.77 |
| 19.8 | 4.48 |
| 20.9 | 4.24 |
| 21.6 | 4.11 |
| 22.8 | 3.89 |
| 24.1 | 3.69 |
| 24.7 | 3.60 |
| 25.4 | 3.50 |
| 26.0 | 3.42 |
| 27.5 | 3.24 |
| 27.7 | 3.21 |
| 28.7 | 3.11 |
| 31.4 | 2.84 |
| 31.6 | 2.83 |
| 32.1 | 2.79 |
| 33.1 | 2.71 |
| 33.6 | 2.66 |
| 34.6 | 2.59 |

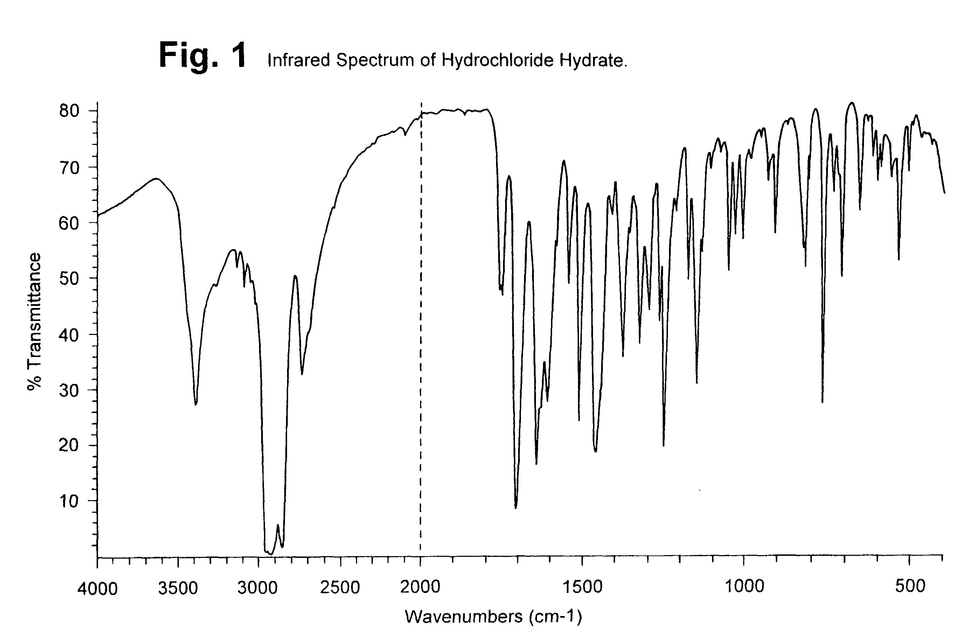

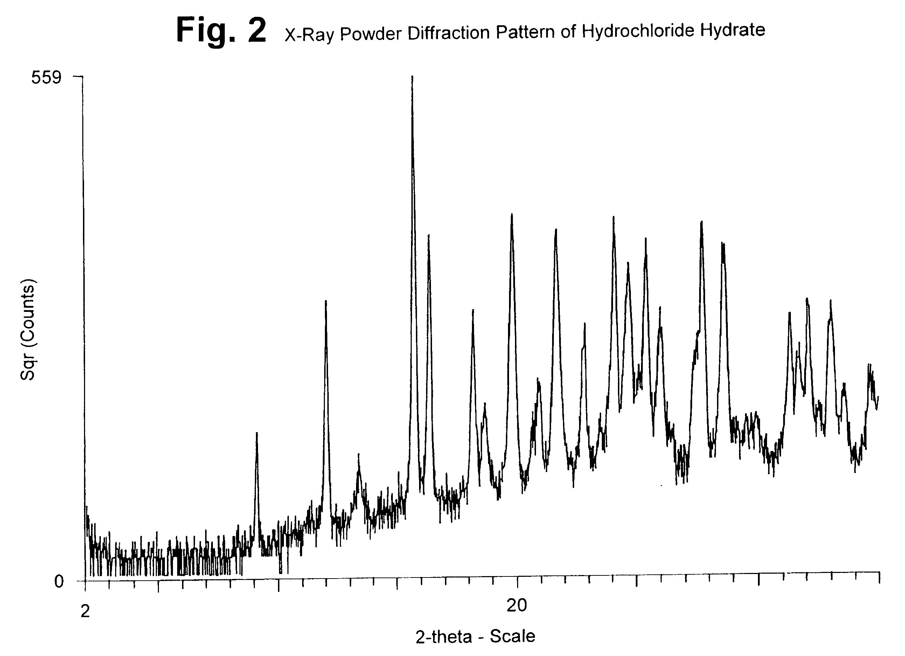

## Claims

1. 5-[4-[2-(N-methyl-N-(2-pyridyl)amino)ethoxy]benzyl]thiazolidine-2,4-dione, hydrochloride dihydrate **characterised in that** it:
(i) provides an infrared spectrum containing peaks at 3392, 2739, 1751, 1325 and 713 cm⁻¹; and/or
(ii) provides an X-ray powder diffraction (XRPD) pattern containing peaks at 9.1, 12.0, 15.7, 16.3 and 19.8 °2θ.

2. A hydrate according to claim 1, which provides an infra red spectrum containing peaks at: 3392, 3139, 3094, 2739, 1758, 1751, 1706, 1643, 1632, 1610, 1583, 1545, 1513, 1412, 1357, 1325, 1297, 1265, 1251, 1216, 1179, 1152, 1138, 1110, 1080, 1053, 1033, 1010, 985, 953, 931, 909, 827, 822, 812, 769, 739, 724, 713, 660, 620, 604, 593, 562, 539, 529 and 508 cm⁻¹.

3. A hydrate according to claim 1 or claim 2, which provides an X-ray powder diffraction (XRPD) pattern containing peaks at 9.1, 12.0,, 15.7, 16.3, 18.2, 18.6, 19.8, 20.9, 21.6, 22.8, 24.1, 24.7, 25.4, 26.0, 27.5, 27.7, 28.7, 31.4, 31.6, 32.1, 33.1, 33.6 and 34.6 °2θ.

4. A hydrate according to any one of claims 1 to 3, in pure form or when admixed with other materials.

5. A hydrate according to claim 4, in pure form.

6. A hydrate according to any one of claims 1 to 5, in crystalline form.

7. A process for preparing a hydrate according to claim 1, **characterised in that** 5-[4-[2-(N-methyl-N-(2-pyridyl)amino)ethoxy]benzyl]thiazolidine-2,4-dione (Compound I) is treated with a suitable source of hydrochloride counter-ion and an appropriate amount of water for formation of the Hydrochloride Hydrate; and thereafter the required compound is recovered.

8. A pharmaceutical composition comprising an effective, non-toxic amount of a hydrate according to claim 1 and a pharmaceutically acceptable carrier therefor.

9. A hydrate according to claim 1, for use as an active therapeutic substance.

10. A hydrate according to claim 1, for use in the treatment and/or prophylaxis of diabetes mellitus, conditions associated with diabetes mellitus and certain complications thereof consisting of renal disease, diabetic nephropathy, glomerulonephritis, glomerular sclerosis, nephrotic syndrome, hypertensive nephrosclerosis and end stage renal disease.

11. The use of hydrate according to claim 1, for the manufacture of a medicament for the treatment and/or prophylaxis of diabetes mellitus, conditions associated with diabetes mellitus and certain complications thereof consisting of renal disease, diabetic nephropathy, glomerulonephritis, glomerular sclerosis, nephrotic syndrome, hypertensive nephrosclerosis and end stage renal disease.

## Patentansprüche

1. 5-[4-[2-(N-Methyl-N-(2-pyridyl)amino)ethoxy]benzyl]thiazolidin-2,4-dion-Hydrochlorid-Dihydrat, **dadurch gekennzeichnet, dass**:
(i) es ein Infrarotspektrum ergibt, das Peaks bei 3392, 2739, 1751, 1325 und 713 cm⁻¹ enthält; und/oder
(ii) es ein Röntgen-Pulverdiffraktionsmuster (XRPD) ergibt, das Peaks bei 9,1, 12,0, 15,7, 16,3 und 19,8° 2θ enthält.

2. Hydrat gemäß Anspruch 1, das ein Infrarotspektrum ergibt, das Peaks bei 3392, 3139, 3094, 2739, 1758, 1751, 1706, 1643, 1632, 1610, 1583, 1545, 1513, 1412, 1357, 1325, 1297, 1265, 1251, 1216, 1179, 1152, 1138, 1110, 1080, 1053, 1033, 1010, 985, 953, 931, 909, 827, 822, 812, 769, 739, 724, 713, 660, 620, 604, 593, 562, 539, 529 und 508 cm⁻¹ enthält.

3. Hydrat gemäß Anspruch 1 oder Anspruch 2, das ein Röntgen-Pulverdiffraktionsmuster (XRPD) ergibt, das Peaks bei 9,1, 12,0, 15,7, 16,3, 18,2, 18,6, 19,8, 20,9, 21,6, 22,8, 24,1, 24,7, 25,4, 26,0, 27,5, 27,7, 28,7, 31,4, 31,6, 32,1, 33,1, 33,6 und 34,6° 28 enthält.

4. Hydrat gemäß einem der Ansprüche 1 bis 3 in Reinform oder gemischt mit anderen Stoffen.

5. Hydrat gemäß Anspruch 4 in reiner Form.

6. Hydrat gemäß einem der Ansprüche 1 bis 5 in kristalliner Form.

7. Verfahren zur Herstellung eines Hydrates gemäß Anspruch 1, **dadurch gekennzeichnet, dass** 5-[4-[2-(N-Methyl-N-(2-pyridyl)amino)ethoxy]benzyl]thiazolidin-2,4-dion (Verbindung I) mit einer geeigneten Hydrochlorid-Gegenionenquelle und einer geeigneten Wassermenge zur Bildung des Hydrochlorid-Hydrates behandelt wird; und danach die gewünschte Verbindung gewonnen wird.

8. Arzneimittel, umfassend eine wirksame, nicht-toxische Menge eines Hydrates gemäß Anspruch 1 und einen pharmazeutisch verträglichen Träger dafür.

9. Hydrat gemäß Anspruch 1, zur Verwendung als therapeutischer Wirkstoff.

10. Hydrat gemäß Anspruch 1, zur Verwendung bei der Behandlung und/oder Prophylaxe von Diabetes mellitus, Beschwerden einhergehend mit Diabetes mellitus und bestimmten Komplikationen davon, bestehend aus Nierenerkrankung, diabetischer Nephropathie, Glomerulonephritis, glomerulärer Sklerose, nephrotischem Syndrom, hypertensiver Nephrosklerose und terminaler Niereninsuffizienz.

11. Verwendung des Hydrates nach Anspruch 1 zur Herstellung eines Medikaments zur Behandlung und/oder Prophylaxe von Diabetes mellitus, Beschwerden einhergehend mit Diabetes mellitus und bestimmten Komplikationen davon, bestehend aus Nierenerkrankung, diabetischer Nephropathie, Glomerulonephritis, glomerulärer Sklerose, nephrotischem Syndrom, hypertensiver Nephrosklerose und terminaler Niereninsuffizienz.

## Revendications

1. Dihydrate de chlorhydrate de 5-[4-[2-(N-méthyl-N-(2-pyridyl)amino)éthoxy]benzyl]thiazolidine-2,4-dione, **caractérisé en ce que** :
(i) il présente un spectre infrarouge contenant des pics à 3392, 2739, 1751, 1325 et 713 cm⁻¹ ; et/ou
(ii) il présente un diagramme de diffraction des rayons X sur poudre (XRPD) contenant des pics à 9,1 ; 12,0 ; 15,7 ; 16,3 et 19,8°2θ.

2. Hydrate suivant la revendication 1, qui présente un spectre infrarouge contenant des pics à : 3392, 3139, 3094, 2739, 1758, 1751, 1706, 1643, 1632, 1610, 1583, 1545, 1513, 1412, 1357, 1325, 1297, 1265, 1251, 1216, 1179, 1152, 1138, 1110, 1080, 1053, 1033, 1010, 985, 953, 931, 909, 827, 822, 812, 769, 739, 724, 713, 660, 620, 604, 593, 562, 539, 529 et 508 cm⁻¹.

3. Hydrate suivant la revendication 1 ou la revendication 2, qui présente un diagramme de diffraction des rayons X sur poudre (XRPD) contenant des pics à : 9,1 ; 12,0 ; 15,7 ; 16,3 ; 18,2 ; 18,6 ; 19,8 ; 20,9 ; 21,6 ; 22,8 ; 24,1 ; 24,7 ; 25,4 ; 26,0 ; 27,5 ; 27,7 ; 28,7 ; 31,4 ; 31,6 ; 32,1 ; 33,1 ; 33,6 ; et 34, 6°2θ.

4. Hydrate suivant l'une quelconque des revendications 1 à 3, sous forme pure ou en mélange avec d'autres substances.

5. Hydrate suivant la revendication 4, sous forme pure.

6. Hydrate suivant l'une quelconque des revendications 1 à 5, sous forme cristalline.

7. Procédé pour la préparation d'un hydrate suivant la revendication 1, **caractérisé en ce que** de la 5-[4-[2-(N-méthyl-N-(2-pyridyl)amino)éthoxy]benzyl]thiazolidine-2,4-dione (Composé I) est traitée avec une source convenable d'ion complémentaire chlorhydrate et une quantité appropriée d'eau pour la formation de l'hydrate de chlorhydrate ; puis le composé requis est recueilli.

8. Composition pharmaceutique comprenant une quantité efficace non toxique d'un hydrate suivant la revendication 1 et un support pharmaceutiquement acceptable à cette fin.

9. Hydrate suivant la revendication 1, destiné à être utilisé comme substance thérapeutique active.

10. Hydrate suivant la revendication 1, destiné à être utilisé dans le traitement et/ou la prophylaxie du diabète sucré, d'affections associées au diabète sucré et de certaines de ses complications consistant en une maladie rénale, la néphropathie diabétique, la glomérulonéphrite, la sclérose glomérulaire, le syndrome néphrotique, la néphrosclérose hypertensive et une maladie rénale au stade final.

11. Utilisation de l'hydrate suivant la revendication 1, pour la production d'un médicament destiné au traitement et/ou à la prophylaxie du diabète sucré, d'affections associées au diabète sucré et de certaines de ses complications consistant en une maladie rénale, la néphropathie diabétique, la glomérulonéphrite, la sclérose glomérulaire, le syndrome néphrotique, la néphrosclérose hypertensive et une maladie rénale au stade final.
